# EUROPEAN PATENT APPLICATION

(11) **EP 3 552 599 A1**
(43) Date of publication of application: **16.10.2019**
(21) Application number: 19168773.0
(22) Date of filing: 11.04.2019
(51) Int. Cl.: A61K 8/73, A61Q 19/00, A61K 8/9794, A61K 8/9783

(54) **COMPOSITION CONTAINING CROCUS SATIVUS EXTRACT AND PREPARATION METHOD AND USE THEREOF**

(30) Priority: 12.04.2018 CN 201810325790
(71) Applicant: Shanghai Traditional Chinese Medicine Co., Ltd., Shanghai 200002 (CN)
(72) Inventor: ZHANG, Xue, Shanghai, Shanghai 200002 (CN); LIU, Guodong, Shanghai, Shanghai 200002 (CN); SI, Ruirong, Shanghai, Shanghai 200002 (CN)
(74) Representative: Martegani, Franco

(57) **Abstract**

The present invention provides a composition containing *Crocus sativus* extract and a preparation method and use thereof. The composition containing *Crocus sativus* extract comprises the following raw materials in parts by mass: 4-6 parts of sodium hyaluronate, 0.5-1.5 parts of *Crocus sativus* extract, 0.5-1.5 parts of *Rosa rugosa* extract, 0.5-1.5 parts of *Lilium candidum* extract, 0.5-1.5 parts of *Calendula officinalis* extract and 0.5-1.5 parts of *Centella asiatica* extract. The facial mask prepared by combining the composition containing *Crocus sativus* extract according to the present invention with Binchotan fiber can deeply cleanse the skin, deeply hydrate the skin, and make the skin clear and bright. After the identification and risk assessment of the safety risk substances in the facial mask, no safety risk was found in the use of this product.

## Description

### FIELD OF THE INVENTION

The present invention relates to a composition containing *crocus sativus* extract and a preparation method and use thereof.

### BACKGROUND OF THE INVENTION

*Crocus sativus* L is one of the raw materials for both medicine and food approved by China's Ministry of Health. Its main medicinal ingredients includes: crocin I and crocin II, no less than 10%; crocin, about 1.5%; crocetin dimethyl ester; picrocrocin, about 2%; and volatile oil, 0.4% to 1.3%. In addition, it also contains compounds such as eucalyptol, pinene, daucosterol, and multivitamins, etc.

*Crocus sativus* L is still widely used in medical fields at home and abroad, and is further widely recognized as a good product for invigorating the circulation of blood and beautifying the skin. Its application mainly includes the following:
Cleaning: The theory of free radical damage suggests that the accumulation of oxygen free radicals produced by aerobic metabolism damages various biological macromolecules and eventually leads to aging. Some antioxidants such as vitamin E, vitamin C, and carotene, etc. can scavenge the free radicals. A facial cleanser containing *Crocus sativus* extract may thoroughly remove dirt in the pores and improve the anti-oxidation and anti-aging abilities of the skin.

Repair: Modern woman bears tremendous pressure from work and life, which may easily cause anxiety, reduced sleep quality, increased blood viscosity, and blockage of capillaries that are invisible to the naked eye. Clinical studies have shown that the hot water extract of *Crocus sativus* has obvious inhibitory effect on blood coagulation, and adenosine, as its active ingredient, can prolong the generation time and activation time of prothrombin and lower the whole blood specific viscosity. A hydrating moisturizing cream containing *Crocus sativus* extract can replenish moisture for skin while shrinking the pores, and in addition, it can further exert its functions of invigorating the circulation of blood and resisting hypoxia, and significantly improve blood microcirculation.

Food: The beautiful appearance of *Crocus sativus* helps increase the color and flavor of cooked foods. Due to carotene, crocin, cis-crocetin dimethyl ester and other active ingredients contained in its stigma, if a stigma of *Crocus sativus* is placed in a glass of water, the full glass of water presents a beautiful golden yellow color, which gives a person with very good visual aesthetic sense while beautifying the skin.

However, when used alone, the *Crocus sativus* extract does not have a long-lasting moisturizing effect and has a poor deep cleansing effect on the skin. Therefore, the development of a facial mask containing *Crocus sativus* extract which can maintain a long-lasting moisturizing effect while having a deep cleaning effect has great commercial value, which is also an urgent problem to be solved in the art.

### SUMMARY OF THE INVENTION

In order to overcome the defects in the prior art that the *Crocus sativus* extract, when used alone, does not have a long-lasting moisturizing effect and has a poor deep cleansing effect on the skin, the present invention provides a composition containing *Crocus sativus* extract and a preparation method and use thereof. The facial mask prepared with the composition containing *Crocus sativus* extract provided by the present invention has the effects of deep cleaning and hydrating.

The present invention solves the above technical problems by the following technical solutions.

The present invention provides a composition containing *Crocus sativus* extract, which comprises the following raw materials in parts by mass: 4-6 parts of sodium hyaluronate, 0.5-1.5 parts of *Crocus sativus* extract, 0.5-1.5 parts of *Rosa rugosa* extract, 0.5-1.5 parts of *Lilium candidum* extract, 0.5-1.5 parts of *Calendula officinalis* extract and 0.5-1.5 parts of *Centella asiatica* extract.

In the present invention, the sodium hyaluronate may be a conventional sodium hyaluronate in the art, preferably purchased from Bloomage Freda Biopharm Co., Ltd.

In the present invention, the *Crocus sativus* extract may be obtained by a conventional method in the art, and is generally obtained by the following method: mixing *Crocus sativus* L with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Crocus sativus* L is produced from a professional planting base for *Crocus sativus* L of Shanghai traditional Chinese medicine Co., Ltd. in Chongming Island.

In the present invention, the *Rosa rugosa* extract may be obtained by a conventional method in the art, and is generally obtained by the following method: mixing *Rosa rugosa* with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Rosa rugosa* extract is purchased from Liaoyuan Daily Chemical Co., Ltd.

In the present invention, the *Lilium candidum* extract may be obtained by a conventional method in the art, and is generally obtained by the following method: mixing *Lilium candidum* with a solvent and then extracting it by a conventional method in the art. The solvent may be a conventional extraction solvent in the art, preferably one or more of water, ethanol and 1, 3-butanediol. The extraction method may be a dipping method, a percolating method, a decocting method, a refluxing method or a continuous extraction method, etc. Preferably, the *Lilium candidum* extract is purchased from Liaoyuan Daily Chemical Co., Ltd.

In the present invention, the *Calendula officinalis* extract may be obtained by a conventional method in the art, and is generally obtained by the following method: mixing *Calendula officinalis* L with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Calendula officinalis* extract is purchased from Liaoyuan Daily Chemical Co., Ltd.

In the present invention, the *Centella asiatica* extract may be obtained by a conventional method in the art, and is generally obtained by the following method: mixing *Centella asiatica* (L.) Urban with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Centella asiatica* extract is purchased from Liaoyuan Daily Chemical Co., Ltd.

In the present invention, the content of the sodium hyaluronate is preferably 5 parts.

In the present invention, the content of the *Crocus sativus* extract is preferably 1 part.

In the present invention, the content of the *Rosa rugosa* extract is preferably 1 part.

In the present invention, the content of the *Lilium candidum* extract is preferably 1 part.

In the present invention, the content of the *Calendula officinalis* extract is preferably 1 part.

In the present invention, the content of the *Centella asiatica* extract is preferably 1 part.

In the present invention, the composition containing *Crocus sativus* extract may further comprise one or more of a solvent, a skin conditioner, a humectant, a thickener, a pH adjuster, a solubilizer, an antioxidant, a preservative, and a fragrance.

Wherein, the solvent may be a conventional one in the art, such as water.

Wherein, the skin conditioner may be a conventional one in the art, such as dipotassium glycyrrhizinate.

Wherein, the content of the skin conditioner may be a conventional content in the art, and the mass ratio of the skin conditioner to the *Crocus sativus* extract is preferably (9 to 11):1, more preferably 10:1.

Wherein, the humectant may be a conventional one in the art, such as propylene glycol.

Wherein, the content of the humectant may be a conventional content in the art, for example, the mass ratio of the humectant to the *Crocus sativus* extract is preferably (1400 to 1600): 1, and more preferably 1500:1.

Wherein, the thickener may be a conventional one in the art, such as hydroxyethyl cellulose.

Wherein, the content of the thickener may be a conventional content in the art, for example, the mass ratio of the thickener to the *Crocus sativus* extract is preferably (30 to 50): 1, and more preferably 40:1.

Wherein, the pH adjuster may be a conventional one in the art, such as triethanolamine.

Wherein, the content of the pH adjuster may be a conventional content in the art, which is a content of the pH adjuster required to adjust to a suitable pH range according to the common knowledge in the art.

Wherein, the solubilizer may be a conventional one in the art, such as PEG-40 hydrogenated castor oil.

Wherein, the content of the solubilizer may be a conventional content in the art, for example, the mass ratio of the solubilizer to the *Crocus sativus* extract is preferably (4 to 6):1, and more preferably 5:1.

Wherein, the antioxidant may be a conventional one in the art, such as tocopheryl acetate.

Wherein, the content of the antioxidant may be a conventional content in the art, for example, the mass ratio of the antioxidant to the *Crocus sativus* extract is preferably (0.5 to 1.5): 1, and more preferably 1:1.

Wherein, the preservative can be a conventional one in the art, such as chlorphenesin.

Wherein, the content of the preservative is in compliance with relevant laws and regulations, and is a conventional content in the art, for example, the mass ratio of the preservative to the *Crocus sativus* extract is preferably (15 to 25):1, and more preferably 20:1.

Wherein, the content of the fragrance may be a conventional content in the art, for example, the mass ratio of the fragrance to the *Crocus sativus* extract is preferably (0.5 to 1.5): 1, and more preferably 1:1.

In a preferred embodiment of the present invention, the composition containing *Crocus sativus* extract comprises the following raw materials in parts by weight: 4-6 parts of sodium hyaluronate, 0.5-1.5 parts of *Crocus sativus* extract, 0.5-1.5 parts of *Rosa rugosa* extract, 0.5-1.5 parts of *Lilium candidum* extract, 0.5-1.5 parts of *Calendula officinalis* extract, 0.5-1.5 parts of *Centella asiatica* extract, 9-11 parts of skin conditioner, 1400-1600 parts of humectant, 30-50 parts thickener, 4-6 parts of solubilizer, 0.5-1.5 parts of antioxidant, 15-25 parts of preservative and 0.5-1.5 parts of fragrance.

In a preferred embodiment of the present invention, the composition containing *Crocus sativus* extract comprises the following raw materials in parts by weight: 5 parts of sodium hyaluronate, 1 part of *Crocus sativus* extract, 1 part of *Rosa rugosa* extract, 1 part of *Lilium candidum* extract, 1 part of *Calendula officinalis* extract and 1 part of *Centella asiatica* extract.

In a preferred embodiment of the present invention, the composition containing *Crocus sativus* extract comprises: 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Rosa rugosa* extract, 0.01% *Lilium candidum* extract, 0.01% *Calendula officinalis* extract, 0.01% *Centella asiatica* extract, 0.1% dipotassium glycyrrhizinate, 0.2% chlorphenesin and 0.01% fragrance, and the above percentages refer to the mass percentage accounting for the total amount of the raw materials.

The present invention also provides a preparation method of the above composition containing *Crocus sativus* extract, in which the raw material components of the composition containing *Crocus sativus* extract are mixed.

When the raw materials contain other component(s) in addition to the sodium hyaluronate, the *Crocus sativus* extract, the *Rosa rugosa* extract, the *Lilium candidum* extract, the *Calendula officinalis* extract and the *Centella asiatica* extract, the other component(s) is/are firstly mixed, and then mixed with "the sodium hyaluronate, the *Crocus sativus* extract, the *Rosa rugosa* extract, the *Lilium candidum* extract, the *Calendula officinalis* extract and the *Centella asiatica* extract".

The present invention also provides use of the above composition containing *Crocus sativus* extract in cosmetics, such as a facial mask.

Wherein, the raw materials of the facial mask are preferably: a solvent, a humectant, a thickener, a pH adjuster, a solubilizer, an antioxidant, sodium hyaluronate, *Crocus sativus* extract, *Rosa rugosa* extract, *Lilium candidum* extract, *Calendula officinalis* extract, *Centella asiatica* extract, a skin conditioner, a preservative, and a fragrance.

The definitions of the solvent, the humectant, the thickener, the pH adjuster, the solubilizer, the antioxidant, the sodium hyaluronate, the *Crocus sativus* extract, the *Rosa rugosa* extract, the *Lilium candidum* extract, the *Calendula officinalis* extract, the *Centella asiatica* extract, the skin conditioner, the preservative and the fragrance are as described above.

The raw materials of the facial mask are preferably the following components: 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Rosa rugosa* extract, 0.01% *Lilium candidum* extract, 0.01% *Calendula officinalis* extract, 0.01% *Centella asiatica* extract, 0.1% dipotassium glycyrrhizinate, 0.2% chlorphenesin and 0.01% fragrance, and the above percentages refer to the mass percentage accounting for the total amount of the raw materials.

Wherein, the substrate of the facial mask is preferably Binchotan fiber.

The Binchotan fiber is preferably made of *Quercus phillyraeoides A.Gary,* and it is known to those skilled in the art that the *Quercus phillyraeoides A.Gary* is in the family Fagaceae.

The preparation method of the Binchotan fiber may be a conventional one in the art, for example, calcining the *Quercus phillyraeoides A.Gary,* grinding it to a nano size, mixing the nano-sized *Quercus phillyraeoides A.Gary* with a plant tree pulp, and then spraying at high pressure. The operations and conditions for calcining may be those commonly used in the art, and the temperature for calcining is preferably 1200 to 1300°C The operations and conditions for grinding may be those commonly used in the art. The plant tree pulp refers to a fibrous material obtained by processing the plant fiber as a raw material. The operations and conditions for spraying at high pressure may be those commonly used in the art, and the pressure for spraying is generally greater than 20 MPa, such as 30 MPa.

In the present application, when the substrate of the facial mask is Binchotan fiber, it can clean the skin, release negative ions, and make the skin clear and bright.

The preparation method of the facial mask includes the following steps:
(1) mixing a group A and a group B, and homogenizing to obtain a mixture 1; the group A includes the humectant, and the group B includes the thickener, the pH adjuster, the sodium hyaluronate, the skin conditioner, and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance;
(4) mixing the mixture 3 in the step (3) with a group D, and homogenizing to obtain a mixture 4; and the group D includes the *Crocus sativus* extract, the *Rosa rugosa* extract, the *Lilium candidum* extract, the *Calendula officinalis* extract, the *Centella asiatica* extract; and
(5) mixing the mixture 4 in the step (4) with the substrate.

In the step (1), the method for mixing the group A and the group B may be a conventional one in the art, for example, mixing the raw materials; preferably, separately adding the raw materials of the group B to the group A; and more preferably, separately adding the raw materials of the group B to the group A after homogenization. The time for homogenizing may be a conventional one in the art, for example, 20 min.

In the step (1), the time for homogenizing is preferably 25 min.

In the step (1), preferably, the mixture 1 is filtered. The mesh number of the filter screen for filtration may be a conventional one in the art, for example, 300 mesh.

In the step (2), the time for homogenizing is preferably 60 to 90 min.

In the step (3), the preparation method of the group C may be a conventional one in the art, for example, uniformly stirring the solubilizer, the antioxidant and the fragrance. The time for stirring may be a conventional one in the art, for example, 10 min.

In the step (3), the time for homogenizing is preferably 25 min.

In the step (4), preferably, the *Crocus sativus* extract, the *Rosa rugosa* extract, the *Lilium candidum* extract, the *Calendula officinalis* extract, the *Centella asiatica* extract are separately added to the mixture 3.

In the step (4), the time for homogenizing is preferably 20 min.

Based on the common knowledge in the art, the above individual preferred conditions can be arbitrarily combined to obtain preferred embodiments of the present invention.

The reagents and raw materials used in the present invention are commercially available.

The beneficial effects of the present invention are that:
(1) The composition containing *Crocus sativus* extract prepared by the present application has a long-lasting effect of resisting oxidation and moisturizing. The prepared *"Crocus sativus* Kishu Binchotan Facial Mask" has a good moisturizing effect, and after applying the facial mask continuously for four weeks and then suspending use for three weeks, the skin may remain the comparable moisture content as it did after one week of use.
(2) The composition containing *Crocus sativus* extract prepared by the present application is combined with Binchotan fiber, as a substrate, to prepare a *"Crocus sativus* Kishu Binchotan Facial Mask", which can deeply cleanse the skin and leave the skin clear and bright.
(3) The preparation process adopted in the present application is simple. Identification and risk assessment were carried out on the safety risk substances of the facial mask, and no safety risk was found in the use of this product.

### DETAILED DESCRIPTION OF THE INVENTION

The invention is further illustrated by the following examples, which are not intended to limit the invention. The experimental methods in the following examples, which do not specify the specific conditions, are selected according to conventional methods and conditions, or according to the manufacturer's instructions.

In the following examples, sodium hyaluronate was purchased from Bloomage Freda Biopharm Co., Ltd.

In the following examples, *Rosa rugosa* extract, *Lilium candidum* extract, *Calendula officinalis* extract and *Centella asiatica* extract were all purchased from Liaoyuan Daily Chemical Co., Ltd.

In the following examples, the preparation method of the *Crocus sativus* extract was: using *Crocus sativus* L produced from the professional planting base for *Crocus sativus* L of Shanghai traditional Chinese medicine Co., Ltd. in Chongming Island, and extracting it according to a conventional method in the art. The extraction solvent may be water and/or ethanol. The extraction method may be a dipping method, a percolating method, a decocting method, a refluxing method or a continuous extraction method, etc.

### Example 1

In this example, the formula of the composition containing *Crocus sativus* extract and the parts by mass of each component were: sodium hyaluronate, 5 parts; *Crocus sativus* extract, 1 part; *Rosa rugosa* extract, 1 part; *Lilium candidum* extract, 1 part; *Calendula officinalis* extract, 1 part; and *Centella asiatica* extract, 1 part.

The composition was prepared by mixing sodium hyaluronate, *Crocus sativus* extract, *Rosa rugosa* extract, *Lilium candidum* extract, *Calendula officinalis* extract, and *Centella asiatica* extract according to their mass ratios.

### Example 2

In this example, the formula of the composition containing *Crocus sativus* extract and the parts by mass of each component were: sodium hyaluronate, 5 parts; *Crocus sativus* extract, 1 part; *Rosa rugosa* extract, 1 part; *Lilium candidum* extract, 1 part; *Calendula officinalis* extract, 1 part; *Centella asiatica* extract, 1 part; and dipotassium glycyrrhizinate, 10 parts.

The composition was prepared by mixing sodium hyaluronate, *Crocus sativus* extract, *Rosa rugosa* extract, *Lilium candidum* extract, *Calendula officinalis* extract, *Centella asiatica* extract, and dipotassium glycyrrhizinate according to their mass ratios.

### Example 3

The raw material formula of a facial mask was: 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Rosa rugosa* extract, 0.01% *Lilium candidum* extract, 0.01% *Calendula officinalis* extract, 0.01% *Centella asiatica* extract, 0.1% dipotassium glycyrrhizinate, 0.2% chlorphenesin and 0.01% fragrance, and the above percentages refer to the mass percentage accounting for the total amount of the raw materials.

The substrate of the facial mask was Binchotan fiber, which was prepared by: calcining the *Quercus phillyraeoides A. Gary,* in the family Fagaceae, at 1200 to 1300°C, grinding it to a nano size, mixing the nano-sized *Quercus phillyraeoides A.Gary* with a plant tree pulp, and then spraying at 30 Mpa.

Preparation method of the facial mask: according to the above mass percentage, propylene glycol was homogenized in a homogenizer for 25 min, then hydroxyethyl cellulose, triethanolamine, sodium hyaluronate, dipotassium glycyrrhizinate and chlorphenesin were added, and the resulting mixture was homogenized for 25 min, and then water was added, and the resulting mixture was homogenized for 90 min, then PEG-40 hydrogenated castor oil, tocopheryl acetate and fragrance were added, and the resulting mixture was homogenized for 25 min, and finally, *Crocus sativus* extract, *Rosa rugosa* extract, *Lilium candidum* extract, *Calendula officinalis* extract and *Centella asiatica* extract were added, and the homogenized mixture was filtered through a 300 mesh filter screen and mixed with Binchotan fiber.

### Effect Example 1

According to the SOD oxidase detection experiment, the composition prepared in Example 1 had a good antioxidant activity, which is more durable than that of the *Crocus sativus* extract alone.

According to the test for the moisture content of skin, the composition prepared in Example 1 had a good moisturizing property, which is more durable than that of the *Crocus sativus* extract alone.

### Effect Example 2

The facial mask prepared in Example 3 may be used in the peel-off mask or may be directly applied to the face.

The indicators of the prepared facial mask are as follows:

### (1) Sensory indicators

Color: transparent; traits: liquid; smell: faint scent; other: none.

### (2) Hygienic chemical indicators

**Table 1 Hygienic chemical indicators**

| Test items | Indicators |
|---|---|
| Mercury | ≤ 1 mg/kg |
| Lead | ≤ 10 mg/kg |
| Arsenic | ≤ 2 mg/kg |
| Cadmium | ≤ 5 mg/kg |
| Dioxane | ≤ 30 mg/kg |

### (3) Microbial indicators

**Table 2 Microbial indicators**

| Test items | Indicators |
|---|---|
| Colony forming unit | ≤ 1000 CFU/g |
| Total count of yeast and mould | ≤ 100 CFU/g |
| Thermotolerant *coliform bacteria* | ND/g |
| *Pseudomonas aeruginosa* | ND/g |
| *Staphylococcus aureus* | ND/g |

### (4) Test methods

**Table 3 Test methods**

| Test items | | Test methods |
|---|---|---|
| | Mercury | Method for the determination of mercury - Method 1 - Hydride atomic fluorescence spectrometry, "Physical and Chemical Testing Methods" in "Safety and Technical Standards for Cosmetics" (2015 Edition). |
| | Arsenic (in arsenic) | Method for the determination of arsenic - Method 1 - Hydride atomic fluorescence spectrometry, "Physical and Chemical Testing Methods" in "Safety and Technical Standards for Cosmetics" (2015 Edition). |
| Hygienic chemical indicators | Lead (in lead) | Method for the determination of lead - Method 1 - Graphite furnace atomic absorption spectrophotometry, "Physical and Chemical Testing Methods" in "Safety and Technical Standards for Cosmetics" (2015 Edition). |
| | Cadmium | Method for the determination of cadmium - Method 1 - Flame atomic absorption spectrophotometry, "Physical and Chemical Testing Methods" in "Safety and Technical Standards for Cosmetics" (2015 Edition). |
| | Dioxane | Method for dioxane: Gas Chromatography-Mass Spectrometry, "Physical and Chemical Testing Methods" in "Safety and Technical Standards for Cosmetics" (2015 Edition). |
| | Colony forming unit | "Microbiological Testing Methods" in "Safety and Technical Standards for Cosmetics" (2015 Edition) |
| | Total count of yeast and mould | "Microbiological Testing Methods" in "Safety and Technical Standards for Cosmetics" (2015 Edition) |
| Microbial indicators | *Fecal coliform* | "Microbiological Testing Methods" in "Safety and Technical Standards for Cosmetics" (2015 Edition) |
| | *Staphylococcus aureus* | "Microbiological Testing Methods" in "Safety and Technical Standards for Cosmetics" (2015 Edition) |
| | *Pseudomonas aeruginosa* | "Microbiological Testing Methods" in "Safety and Technical Standards for Cosmetics" (2015 Edition) |

### (5) How to use

After cleansing, the facial mask was spread over the face and smoothed by hands.

### (6) Safety assessment of risk substances

**Table 4**

| Serial number of raw materials | Standard Chinese name of raw materials | Is there a safety risk substance? | Remark |
|---|---|---|---|
| 1 | Water | × | No safety risk substance was detected. |
| 2 | Propylene glycol | × | No safety risk substance was detected. |
| 3 | Hydroxyethyl cellulose | × | No safety risk substance was detected. |
| 4 | Triethanolamine | Secondary alkaneamine, nitrosamine | It met the relevant requirements in the limited substances in Table 3 of the "Safety and Technical Standards for Cosmetics" (2015 Edition). For details, please refer to the raw material specification of triethanolamine. |
| 5 | PEG-40 hydrogenated castor oil | Dioxane | The residual amount of dioxane was in accordance with the requirement that the limit value of dioxane is not more than 30 mg/kg in "Safety and Technical Standards for Cosmetics" (2015 Edition). For details, please refer to the raw material specification or the test report of the final product. |
| 6 | Sodium hyaluronate | × | No safety risk substance was detected. |
| 7 | Tocopherol acetate | × | No safety risk substance was detected. |
| 8 | *Crocus sativus* extract | × | No safety risk substance was detected. |
| 9 | *Rosa rugosa* extract | × | No safety risk substance was detected. |
| 10 | *Lilium candidum* extract | × | No safety risk substance was detected. |
| 11 | *Calendula officinalis* extract | × | No safety risk substance was detected. |
| 12 | *Centella asiatica* extract | × | No safety risk substance was detected. |
| 13 | Dipotassium glycyrrhizinate | × | No safety risk substance was detected. |
| 14 | Chlorphenesin | Chlorphenesin | The amount of chlorpheniramine added in this product was 0.2%, <0.3%, which met the requirements for chlorphenesin in "Safety and Technical Standards for Cosmetics" (2015 Edition). |
| 15 | Fragrance | × | No safety risk substance was detected. |

### (7) Instant use effect of the facial mask

Twenty volunteers aged between 30 and 40 years old were selected. After facial cleansing, they used the facial mask prepared in Example 3 of this application. The scores after use are as follows.

**Table 5**

| | |
|---|---|
| 5 points | There is no dirt in the furrows of the skin, and the skin is delicate and smooth, clear and bright, soft and silky. |
| 4 points | There is no obvious dirt in the furrows of the skin, and the skin is smooth and plump. |
| 3 points | The skin is smooth. |
| 2 points or less | No significant improvement on skin is found. |

**Table 6**

| | |
|---|---|
| 5 points | 18 |
| 4 points | 2 |
| 3 points | 0 |
| 2 points or less | 0 |

It can be seen from Table 5 and Table 6 that the facial mask prepared in Example 3 of this application had a good skin cleansing and moisturizing performance, and the score rate of 5 points reached 90%.

### (8) Long-term use effect of the facial mask

Experiment on long-term moisturizing effect on facial skin: Twenty women aged between 30 and 40 were selected, who separately used the facial mask product prepared in Example 3 and a certain brand of hydrating cosmetic purchased on the market (as Comparative Example 1). The facial mask was applied once a day for the first three days, and then once every two days, for four weeks. Before use, the moisture content of skin was measured with a skin tester under controlled conditions of a constant temperature of 25°C and a relative humidity of 40% as a reference. The moisture content of skin was measured after one week, two weeks, and four weeks of use, and three weeks after the suspension of use. The results are shown in the table below.

**Table 7**

| | One week | Two weeks | Four weeks | After three weeks of suspension |
|---|---|---|---|---|
| Example 3 | 45 | 48 | 50 | 46 |
| Comparative Example 1 | 30 | 32 | 33 | 10 |

As can be seen from Table 7, based on the values measured by the skin tester before the experiment, the moisture content of skin was significantly increased after a predetermined period of use. Compared with the certain brand of hydrating cosmetic, the facial mask prepared by this application had better moisturizing performance. Moreover, even after three weeks of suspension, the skin remained the equivalent moisture content as it did after one week of use, indicating that the facial mask prepared in Example 3 has a long-lasting moisturizing property.

The facial mask prepared by the composition formulae of Example 1 and Example 2 had an effect comparable to that of Example 3.

## Claims

1. A composition containing *Crocus sativus* extract, comprising the following raw materials in parts by mass: 4-6 parts of sodium hyaluronate, 0.5-1.5 parts of *Crocus sativus* extract, 0.5-1.5 parts of *Rosa rugosa* extract, 0.5-1.5 parts of *Lilium candidum* extract, 0.5-1.5 parts of *Calendula officinalis* extract and 0.5-1.5 parts of *Centella asiatica* extract.

2. The composition of claim 1, wherein the content of the sodium hyaluronate is 5 parts;
and/or, the content of the *Crocus sativus* extract is 1 part;
and/or, the content of the *Rosa rugosa* extract is 1 part;
and/or, the content of the *Lilium candidum* extract is 1 part;
and/or, the content of the *Calendula officinalis* extract is 1 part;
and/or, the content of the *Centella asiatica* extract is 1 part.

3. The composition of claim 1 or 2, wherein the composition further comprises one or more of a solvent, a skin conditioner, a humectant, a thickener, a pH adjuster, a solubilizer, an antioxidant, a preservative, and a fragrance.

4. The composition of claim 3, wherein the composition further comprises a solvent, and the solvent is water;
the composition further comprises a skin conditioner, and the skin conditioner is dipotassium glycyrrhizinate; and the mass ratio of the skin conditioner to the *Crocus sativus* extract is (9 to 11):1, and preferably 10:1.
the composition further comprises a humectant, and the humectant is propylene glycol; and the mass ratio of the humectant to the *Crocus sativus* extract is (1400 to 1600): 1, and preferably 1500:1;
the composition further comprises a thickener, and the thickener is hydroxyethyl cellulose; and the mass ratio of the thickener to the *Crocus sativus* extract is (30 to 50): 1, and preferably 40:1;
the composition further comprises a pH adjuster, and the pH adjuster is triethanolamine;
the composition further comprises a solubilizer, and the solubilizer is PEG-40 hydrogenated castor oil; and the mass ratio of the solubilizer to the *Crocus sativus* extract is (4 to 6):1, and preferably 5:1;
the composition further comprises an antioxidant, and the antioxidant is tocopheryl acetate; and the mass ratio of the antioxidant to the *Crocus sativus* extract is (0.5 to 1.5): 1, and preferably 1:1;
the composition further comprises a preservative, and the preservative is chlorphenesin; and the mass ratio of the preservative to the *Crocus sativus* extract is (15 to 25): 1, and preferably 20:1; and
the composition further comprises a fragrance, and the mass ratio of the fragrance to the *Crocus sativus* extract is (0.5 to 1.5): 1, and preferably 1:1.

5. The composition of claim 1, wherein the composition comprises the following raw materials in parts by weight: 4-6 parts of sodium hyaluronate, 0.5-1.5 parts of *Crocus sativus* extract, 0.5-1.5 parts of *Rosa rugosa* extract, 0.5-1.5 parts of *Lilium candidum* extract, 0.5-1.5 parts of *Calendula officinalis* extract, 0.5-1.5 parts of *Centella asiatica* extract, 9-11 parts of skin conditioner, 1400-1600 parts of humectant, 30-50 parts thickener, 4-6 parts of solubilizer, 0.5-1.5 parts of antioxidant, 15-25 parts of preservative and 0.5-1.5 parts of fragrance.

6. The composition of claim 1, wherein, the composition comprises the following raw materials in parts by weight: 5 parts of sodium hyaluronate, 1 part of *Crocus sativus* extract, 1 part of *Rosa rugosa* extract, 1 part of *Lilium candidum* extract, 1 part of *Calendula officinalis* extract and 1 part of *Centella asiatica* extract.

7. The composition of claim 1, wherein, the composition comprises: 84.12% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Crocus sativus* extract, 0.01% *Rosa rugosa* extract, 0.01% *Lilium candidum* extract, 0.01% *Calendula officinalis* extract, 0.01% *Centella asiatica* extract, 0.1% dipotassium glycyrrhizinate, 0.2% chlorphenesin and 0.01% fragrance, and the above percentages refer to the mass percentage accounting for the total amount of the raw materials.

8. A preparation method of the composition of any one of claims 1 to 7, wherein the raw material components of the composition are mixed.

9. The preparation method of claim 8, wherein the raw materials contain other component(s) in addition to the sodium hyaluronate, the *Crocus sativus* extract, the *Rosa rugosa* extract, the *Lilium candidum* extract, the *Calendula officinalis* extract and the *Centella asiatica* extract, and the other component(s) is/are firstly mixed and then mixed with the sodium hyaluronate, the *Crocus sativus* extract, the *Rosa rugosa* extract, the *Lilium candidum* extract, the *Calendula officinalis* extract and the *Centella asiatica* extract.

10. The composition of any one of claims 1 to 7 for use in cosmetics.

11. The composition for the use of claim 10, wherein the cosmetic is a facial mask, and the raw materials of the facial mask are: a solvent, a humectant, a thickener, a pH adjuster, a solubilizer, an antioxidant, sodium hyaluronate, *Crocus sativus* extract, *Rosa rugosa* extract, *Lilium candidum* extract, *Calendula officinalis* extract, *Centella asiatica* extract, a skin conditioner, a preservative, and a fragrance; and
the substrate of the facial mask is Binchotan fiber made of *Quercus phillyraeoides A.Gary.*

12. The composition for the use of claim 11, wherein the Binchotan fiber is prepared by calcining the *Quercus phillyraeoides A.Gary,* grinding to a nano size, mixing the nano-sized *Quercus phillyraeoides A.Gary* with a plant tree pulp, and then spraying at high pressure.

13. The composition for the use of claim 12, wherein the temperature for calcining is 1200 to 1300°C, and the pressure for spraying is greater than 20 MPa.

14. The composition for the use of claim 11, wherein the preparation method of the facial mask includes the following steps:
(1) mixing a group A and a group B, and homogenizing to obtain a mixture 1; the group A includes the humectant, and the group B includes the thickener, the pH adjuster, the sodium hyaluronate, the skin conditioner, and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance;
(4) mixing the mixture 3 in the step (3) with a group D, and homogenizing to obtain a mixture 4; and the group D includes the *Crocus sativus* extract, the *Rosa rugosa* extract, the *Lilium candidum* extract, the *Calendula officinalis* extract, the *Centella asiatica* extract; and
(5) mixing the mixture 4 in the step (4) with Binchotan fiber.

15. The composition for the use of any one of claims 11 to 14, wherein the solvent is water;
the skin conditioner is dipotassium glycyrrhizinate; and the mass ratio of the skin conditioner to the *Crocus sativus* extract is (9 to 11):1, and preferably 10:1;
the humectant is propylene glycol; and the mass ratio of the humectant to the *Crocus sativus* extract is (1400 to 1600): 1, and preferably 1500:1;
the thickener is hydroxyethyl cellulose; and the mass ratio of the thickener to the *Crocus sativus* extract is (30 to 50): 1, and preferably 40:1;
the pH adjuster is triethanolamine;
the solubilizer is PEG-40 hydrogenated castor oil; and the mass ratio of the solubilizer to the *Crocus sativus* extract is (4 to 6):1, and preferably 5:1;
the antioxidant is tocopheryl acetate; and the mass ratio of the antioxidant to the *Crocus sativus* extract is (0.5 to 1.5): 1, and preferably 1:1;
the preservative is chlorphenesin; and the mass ratio of the preservative to the *Crocus sativus* extract is (15 to 25): 1, and preferably 20:1; and
the mass ratio of the fragrance to the *Crocus sativus* extract is (0.5 to 1.5): 1, and preferably 1:1.
